# NEUE EUROPÄISCHE PATENTSCHRIFT

(11) **EP 1 098 700 B2**
(45) Veröffentlichungstag und Bekanntmachung des Hinweises auf die Entscheidung über den Einspruch: **24.12.2014**
(45) Hinweis auf die Patenterteilung: 16.10.2002
(21) Anmeldenummer: 99942719.8
(22) Anmeldetag: 01.07.1999
(51) Int. Cl.: B01J 21/16, B01J 37/10, C07C 29/04, B01J 37/06

(54) **VERFAHREN ZUR HERSTELLUNG EINES ENTALUMINIERTEN KATALYSATORS/KATALYSATORTRAGERS**
METHOD FOR PRODUCING A DEALUMINIZED CATALYST
PROCEDE DE PRODUCTION D`UN CATALYSEUR DESALUMINISE

(30) Priorität: 03.07.1998 DE 19829747
(43) Veröffentlichungstag der Anmeldung: 16.05.2001
(73) Patentinhaber: Sasol Solvents Germany GmbH, 20537 Hamburg (DE)
(72) Erfinder: SAKUTH, Michael, D-45772 Marl (DE); LOHRENGEL, Gregor, D-46286 Dorsten (DE); MASCHMEYER, Dietrich, D-45657 Recklinghausen (DE); STOCHNIOL, Guido, D-45770 Marl (DE)
(74) Vertreter: King, Alex
(86) Internationale Anmeldenummer: PCT/DE1999/001898
(87) Internationale Veröffentlichungsnummer: WO 2000/001480

(56) Entgegenhaltungen:
- EP-A- 0 283 649
- EP-A- 0 503 229
- EP-A1- 1 028 804
- EP-A2- 0 578 441
- DE-B- 1 156 772
- DE-B- 1 193 928
- GB-A- 1 306 141

## Beschreibung

Beansprucht wird ein Verfahren zur Herstellung eines entaluminierten Katalysators / Katalysatorträgers.

Bekannt ist, daß sich lineare oder gering verzweigte Olefine niederer Molekülmasse in der Gasphase bei erhöhten Drücken und Temperaturen mit Wasserdampf zu Alkoholen umsetzen lassen. Von großtechnischer Bedeutung ist dabei die Synthese von Ethanol aus Ethen und Isopropanol aus Propen. Die Herstellung dieser Alkohole erfolgt in Gegenwart saurer Katalysatoren, wobei in der Regel ein mit Phosphorsäure getränktes, alumosilikatisches bzw. silikatisches Material als Katalysatorträger verwendet wird.

Das Material des Katalysatorträgers baut sich entweder aus reiner Kieselsäure auf, wie beispielsweise Kieselgel (US 2 579 601), oder besteht aus Kieselsäure mit variierenden Tonerdegehalten (US 3 311 568) bzw. aus reinen, beispielsweise montinorillonithaltigen Schichtsilikaten (DE 29 08 491).

Neben diesen phoshorsäurehaltigen Katalysatorträgern werden auch zeolithische Materialien (EP 0 323 269 B1) oder andere saure Katalysatoren, wie z.B. Zirkonphosphate (GB 005 534), verwendet.

Bei den Trägern, die ausschließlich auf Kieselsäure in Form von Kieselgelen basieren, ist die mechanische Festigkeit über eine längere Standzeit bisher problematisch. Aluminiumhaltige Katalysatorträger oder solche, die nur auf Tonerden basieren, zeigen zwar eine deutlich höhere Langzeitstabilität, besitzen aber den erheblichen

Nachteil, daß während der Hydratisierungsreaktion Aluminium durch die Einwirkung der Phosphorsäure aus dem Katalysatorträger herausgelöst wird. Das Aluminium findet sich als schwerlösliche Ablagerung in Form von Aluminiumphosphaten in den nachgeschalteten Apparaten wieder. Diese werden hierdurch allmählich verblockt.

In DE 1 156 772 wird ein Verfahren vorgestellt, den Aluminiumgehalt der Schichtsilikate durch die Einwirkung von Salzsäure zu senken. Jedoch weist das Trägermaterial selbst bei intensiver Salzsäurewäsche noch einen Restaluminiumgehalt von etwa 1 bis 2 Gew.-% auf.

In EP 0 578 441 B1 wird mit einem pelletisierten Silikatträger auf Aerosilbasis (Degussa), der kein Aluminium enthält, eine gewisse Langzeitstabilität erreicht. Ausgangsmaterial für die Herstellung von Aerosil ist das relativ teure Siliziumtetrachlorid. Da es sich bei Materialien auf Basis von Schichtsilikaten, wie beispielsweise dem Montmorillonit, um ein Naturprodukt handelt, das in entsprechenden Lagerstätten abgebaut werden kann, besitzen diese gegenüber pelletisierten Silikatträgern einen deutlichen Vorteil in der Wirtschaftlichkeit des Hydratisierungsprozesses.

Der vorliegenden Erfindung liegt daher die Aufgabe zugrunde ein Verfahren zur Herstellung eines Katalysators für ein wirtschaftliches Verfahren zur Hydratisierung von C₂- oder C₃- Olefinen, der aus einem mit Säure getränkten Katalysatorträger besteht, zu finden, bei welchem der Katalysatorträger eine möglichst hohe Langzeitstabilität besitzt und gleichzeitig der Austrag an Aluminium während der Hydratisierungsreaktion möglichst gering ist.

Überraschenderweise wurde gefunden, daß ein entaluminierter Katalysatorträger, auf Basis von im wesentlichen aluminiumhaltigen Schichtsilikaten mit Montmorillonit-Struktur, mit einem Aluminiumgehalt kleiner 0,03 Gew.-% eine hohe Langzeitstabilität aufweist, und daß bei einem Verfahren zur Hydratisierung von C₂- oder C₃- Olefinen mit Wasser in Gegenwart eines Katalysators, der aus einem mit Säure getränkten

Katalysatorträger besteht, durch Durchführen der Hydratisierungsreaktion mit einem entaluminierten Katalysatorträger keine oder nur geringe Mengen Aluminium aus dem Katalysatorträger ausgewaschen werden.

Gegenstand der vorliegenden Erfindung ist deshalb ein Verfahren zur Herstellung eines entaluminierten Katalysators / Katalysatorträgers, auf Basis von im wesentlichen aluminiumhaltigen Schichtsilikaten mit Montmorillonit-Struktur, mit einem Aluminiumgehalt kleiner 0,03 Gew.-%.

Nach dem erfindungsgemäßen Verfahren ist der Katalysatorträger mit verringertem Aluminiumgehalt, der im wesentlichen aluminiumhaltige Schichtsilikate mit Montmorillonit-Struktur umfaßt, erhältlich, indem man den Katalysatorträger
- mit Phosphorsäure tränkt,
- hydrothermal bei einer Temperatur von 160 bis 300 °C und einem Wasserdampfpartialdruck von 4 bis 80 bar_{absolut} behendelt,
- nachfolgend mit saurer, basischer oder neutraler Lösung bei einer Temperatur von 20 bis 100 °C wäscht und
- anschließend mit Wasser bis zur Neutralität des Waschwassers nachwäscht.

Der nach dem erfindungsgemäßen Verfahren erhältliche mit Säure getränkte Katalysator kann zur Hydratisierung von C₂- oder C₃,- Olefinen mit Wasser eingesetzt werden.

Unter Hydratisierung bzw. Hydratisierungsreaktionen wird die Reaktion von Wasser mit einer Kohlenstoff-Kohlenstoff- Doppelbindung verstanden.

Unter Entaluminierung bzw. entaluminierten Katalysatorträgern wird im Sinne der vorliegenden Erfindung ein Verfahren zur Verringerung des Aluminiumgehalts bzw. ein Katalysatorträger mit einem verringertem Aluminiumgehalt verstanden.

Durch Einsatz des erfindungsgemäßen Verfahrens kann ein auf Basis von kalzinierten und nachbehandelten Schichtsilikaten basierender Katalysatorträger hergestellt werden, der einen deutlich geringeren Gehalt an Aluminium aufweist als ein nicht erfindungsgemäß behandelter Katalysatorträger. Trotz des geringeren Aluminiumgehalts behält der Katalysator seine Langzeitstabilität. Durch Einsatz des erfindungsgemäß hergestellten Katalysatorträgers in dem Verfahren zur Hydratisierung von C₂- oder C₃- Olefinen mit Wasser wird der Anteil an während der Hydratisierungsreaktion ausgewaschenem Aluminium deutlich verringert. Dadurch entstehen während der Hydratisierungsreaktion weniger schwer lösliche Aluminiumverbindungen, die bei herkömmlichen Verfahren die Standzeiten nachgeschalteter Apparaturen, wie z.B. Wärmetauscher, verringern, indem sie Leitungen oder Wärmetauscherflächen zusetzen.

Der nach dem erfindungsgemäßen Verfahren hergestellte entaluminierte Katalysatorträger mit einem Aluminiumgehalt kleiner 0,03 Gew.-%, enthält im wesentlichen aluminiumhaltige Schichtsilikate. Die aluminiumhaltigen Schichtsilikate sind vorzugsweise Smektite und weisen vorzugsweise Montmorillonit-Struktur auf. Schichtsilikate, welche im wesentlichen aluminiumhaltige Schichtsilikate mit Montmorillonit-Struktur aufweisen, sind z.B. die Bentonite. Neben den Montmorilloniten können die Bentonite als weitere Bestandteile z.B. Glimmer, Illit, Cristobalit und Zeolithe enthalten.

Ausgangsstoff für die Herstellung des Katalysatorträgers sind handelsübliche Katalysatortrager z.B. auf Basis von kalzinierten und nachbehandelten Schichtsilikaten.

Nach dem erfindungsgemäßen Verfahren wird ein entaluminierter Katalysatorträger mit einem Aluminiumgehalt kleiner 0,3 Gew.-%, vorzugsweise kleiner 0,03 Gew.-%, auf Basis von im wesentlichen aluminiumreichen Schichtsilikaten mit Montmorillonit-Struktur durch Tränken des Katalysatorträgers mit Phosphorsäure, vorzugsweise einer 10 bis 90 Gew.-%igen Phosphorsäure hergestellt, besonders bevorzugt einer 50 bis 60 Gew.-%igen Phosphorsäure, so daß der Katalysatorträger 5 bis 60 %, vorzugsweise 30 bis 40 % Phosphorsäure enthält, darauffolgendes hydrothermales Behandeln bei einer Temperatur von 160 bis 300 °C, vorzugsweise bei einer Temperatur von 220 bis 260°C und einem Wasserdampfpartialdruck von 4 bis 80. bar_{absolut}, vorzugsweise bei einem Wasserdampfpartialdruck von 16 bis 25 bar_{absolut}, nachfolgender Wäsche mit saurer, basischer oder neutraler, vorzugsweise saurer oder neutraler Lösung, besonders bevorzugt mit Wasser, Salzsäure oder Wasser; welches 0 bis 30 Teile konzentrierte Salzsäure enthält; bei einer Temperatur von 20 bis 100 °C, vorzugsweise von 70 bis 90 °C und anschließendes Nachwaschen des Katalysatorträgers bis zur Neutralität des Waschwassers erhalten werden.

Eine beispielhafte Ausführungsart des erfindungsgemäßen Verfahrens zur Verringerung des Aluminiumgehalts eines Katalysatorträgers wird im folgenden beschrieben, ohne daß das erfindungsgemäße Verfahren auf diese beschränkt ist.

Zur Verringerung des Aluminiumgehalts eines Katalysatorträgers, der im wesentlichen aluminiumhaltige Schichtsilikate umfaßt, können handelsübliche, Schichtsilikate, wie z.B. Montmorillonite oder Bentonite, enthaltende Katalysatorträger verwendet werden. Die Katalysatorträger haben vorzugsweise die Form von sphärischen Körpern, wie z.B. Kugeln, Linsen, Quadern, Zylindern oder auch unregelmäßige Formen, besonders bevorzugt haben sie die Form von Kugeln. Die sphärischen Körper weisen vorzugsweise einen durchschnittlichen Durchmesser von 1 bis 10 mm, besonders bevorzugt einen von 4 bis 6 mm, auf.

Zur Verringerung des Aluminiumgehaltes im Katalysatorträger wird der Katalysatorträger in Säure getränkt, hydrothermal behandelt, anschließend gewaschen und schließlich nachgewaschen.

Der Katalysatorträger wird zum Erzielen des erfindungsgemäßen Effekts in Säure, vorzugsweise Phosphorsäure getränkt. Es wird eine 10 bis 90 Gew.-%ige Phosphorsäure, vorzugsweise eine 50 bis 60 Gew.-%ige Phosphorsäure verwendet. Nach dem Tränken sollte der Katalysatorträger einen Gehalt an Phosphorsäure von 5 bis 60 Gew.-%, vorzugsweise 30 bis 40 Gew.-%, aufweisen. Der Katalysatorträger wird anschließend hydrothermal behandelt.

Bei den hydrothermalen Bedingungen wandeln sich Schichtsilikatmaterialien, wie z.B. Montmorillonit in cristobalitähnliche Strukturen um. Einhergehend verschwinden die ursprünglich vorhandenen Mikroporen. Diese morphologischen Strukturveränderungen zeigen sich deutlich in der BET-Oberfläche, dem Porenvolumen und der Porenradienverteillung Unter den hydrothermalen Reaktionsbedingungen gelangt man zu sogenannten "offenen" Porenstrukturen.

Die hydrothermale Behandlung des schichtsilikathaitigen Katalysatorträgers kann bei Temperaturen zwischen 160 und 300 °C und einem Wasserdampfpartialdruck zwischen 4 und 80 bar_{absolut}, bevorzugt zwischen 220 und 260 °C und einem Wasserdampfpartialdruck von 16 bis 25 bar_{absolut}, erfolgen

Nach der hydrothermalen Behandlung wird der Katalysatorträger mit einer basischen, sauren oder neutralen Lösung, vorzugsweise mit einer sauren oder neutralen Lösung, besonders bevorzugt mit Salzsäure, mit Wasser, welches 0 bis 30 Teile konzentrierte Salzsaure enthält oder mit einer neutralen wäßrigen Lösung, gewaschen. Das Waschen des Katalysatorträgers wird bei einer Temperatur von 20 bis 100 °C, vorzugsweise von 70 bis 90 °C, durchgeführt.

Nach dem Waschen kann der Katalysatorträger mit Wasser bis zur Neutralität des Waschwassers nachgewaschen werden. Die Katalysatorträger weisen ein Gesamtporenvolumen von 0,2 bis 0,9 ml/g, besonders bevorzugt zwischen 0,6 und 0,7 ml/g, auf. Die Druckfestigkeit der Katalysatorträger sollte zumindest 10 N/mm, vorzugsweisezumindest 20 N/mm betragen.

In einer besonderen Ausführungsart des erfindungsgemäßen Verfahren erfolgt die hydrothermale Behandlung des mit Säure getränkten Katalysatorträgers, der 5 bis 60 Gew.-%, vorzugsweise 30 bis 40 Gew.-%, Phosphorsäure enthält, durch Einsatz als Katalysator in einer Hydratisierungsreaktion, von C₂- oder C₃- Olefinen. Zum Tränken des Katalysatorträgers wird vorzugsweise eine 10 bis 90 Gew.-%ige, besonders bevorzugt eine 50 bis 60 Gew.-%ige, Phosphorsäure verwendet.

Bei dieser Hydratisierungsreaktion wird in einem mit dem Katalysator gefüllten Reaktor, vorzugsweise einem Rohrreaktor, Olefin und Wasser einem Molverhältnis von 0,1 bis 0,8, vorzugsweise von 0,15 und 0,5 zur Reaktion gebracht. Das eingesetzte Olefin und das eingesetzte Wasser werden gasförmig oder flüssig, vorzugsweise gasförmig, in den Reaktor gefahren. Zum Verdampfen des Wassers bzw zum Heizen beider Edukte auf Reaktionstemperatur kann es vorteilhaft sein, beide Edukte über eine Verdampferund/oder Thermostatisierungsstrecke, die elektrisch oder mittels Wärmeträger auf Reaktionstemperatur beheizt sind, in den Reaktor zu führen. Die Gas-Hourly-Space-Velocity (GHSV) sollte zwischen 10 und 100 lₙ/min/l_{Kat} betragen. Die Hydratisierungsreaktion von wird bei einer Temperatur von 160 bis 300 °C und einem Druck von 20 bis 200 bar_{absolut}, ausgeführt. Die Hydratisierung von Ethen zu Ethanol wird vorzugsweise bei einer Temperatur von 220 bis 260 °C und einem Druck von 60 bis 80 bar_{absolut} durchgeführt.

Der Reaktorausgang kann vorzugsweise mit einem Kühler verbunden sein, der einen Großteil der unterkritischen Komponenten, auskondensiert und diese weiteren Aufarbeitungsschritten, z.B. einer destillativen Trennung, zugänglich macht.

Zur Kontrolle der Aktivität und Selektivität des mit Säure getränkten Katalysatorträgers kann es vorteilhaft sein, den Austrittsstrom aus dem Reaktor zu analysieren. Die Analyse kann z.B. gaschromatographisch erfolgen.

Zur Verlängerung der Standzeit des Katalysators kann es vorteilhaft sein, kontinuierlich oder diskontinuierlich, vorzugsweise kontinuierlich die Säure mit der der Katalysatorträger getränkt wurde, in den Reaktor nachzudosieren. Das Einbringen der Säure in den Reaktor kann z.B. durch Eindüsen erfolgen. Die Menge der Säure, die in den Reaktor eingebracht wird,- kann von dem Analyseergebnis des Austrittsstroms abhängig gemacht werden. Die Analyse des Austrittstroms und die Bestimmung der daraus resultierenden Säuremenge, die zugegeben wird, kann automatisch erfolgen.

Nach der hydrothermalen Behandlung des Katalysatorträgers, durch Einsatz als Katalysator in einer Hydratisierungsreaktion, wird die Restsäure, mit' welcher der Katalysatorträger getränkt wurde, durch Waschen mit Wasser bis zur Neutralität des Waschwassers entfernt.

Nach dem Entfernen der Restsäure wird der Katalysatorträger mit einer basischen, sauren oder neutralen Lösung, vorzugsweise mit einer sauren oder neutralen Lösung, besonders bevorzugt mit Salzsäure, mit Wasser, welches 0 bis 30 Teile konzentrierte Salzsäure enthält oder mit einer neutralen wäßrigen Lösung, gewaschen. Der Katalysatorträger kann bei einer Temperatur von 20 bis 100 °C, vorzugsweise bei einer Temperatur von 70 bis 90 °C gewaschen werden.

Nach dem Waschen kann der Katalysatorträger mit Wasser bis zur Neutralität des Waschwassers nachgewaschen werden.

Bei Katalysatorträgern, die durch Verwendung als Katalysator in einer Hydratisierungsreaktion hydrothermal behandelt wurden, kann es vorteilhaft sein, nach dem Verringern des Aluminiumgehaltes im Katalysatorträger, den Katalysatorträger durch Abbrennen eventuell anhaftender kohlenstoffhaltiger Verbindungen bei 300 bis 1000°C, vorzugsweise bei 450 bis 500 °C, zu reinigen.

Bei beiden Ausführungsarten des erfindungsgemäßen Verfahrens erhält man einen behandelten Katalysatorträger mit einem verringerten Gehalt an Aluminium. Die behandelten Katalysatorträger haben einen durchschnittlichen Durchmesser von 1 bis 10 mm, bevorzugt einen von 4 bis 6 mm. Das Gesamtporenvolumen beträgt von 0,27 bis 0,9 ml/g, vorzugsweise von 0,6 bis 0,7 ml/g. Die Druckfestigkeit nach der Behandlung des Katalysatorträgers beträgt zumindest 10 N/mm, vorzugsweise zumindest 20 N/mm. Der Aluminiumgehalt der behandelten Kalalysatorträger ist kleiner 0,3 Gew.%, vorzugsweise kleiner 0,03 Gew.-%.

Die nach dem erfindungsgemäßen Verfahren hergestellten Katalysatorträger mit verringertem Aluminiumgehalt können zur Herstellung von Katalysatoren verwendet werden.

Die nach dem erfindungsgemäßen Verfahren hergestellten Katalysatorträger mit verringertem Aluminiumgehalt können erfindungsgemäß zur Hydratisierung von C₂- oder C₃- Olefinen mit Wasser in Gegenwart eines Katalysators, der aus einem mit Säure getränkten, erfindungsgemäß behandelten Katalysatorträger besteht, eingesetzt werden.

Der Phosphorsäuregehalt des getränkten Katalysatorträgers -sollte zum Erreichen einer maximalen Katalysätoraktivität zwischen 5 bis 60 Gew.-% liegen, bevörzugt zwischen 30 bis 40 Gew.-%. Zum Tränken des Katalysatorträgers wird eine wäßrige Phosphorsäurelösung mit einem Phosphorsäuregehalt von 10 bis 90 Gew.-%, vorzugsweise von 50 bis 60 Gew.-% eingesetzt. Der so hergestellte saure Katalysator wird in einen Reaktor, vorzugsweise einen Rohrreaktor, gefüllt. Der Reaktor wird isotherm oder nicht isotherm, vorzugsweise isotherm, betrieben und kann elektrisch oder mittels Wärmeträgern beheizt werden.

Der Reaktor wird kontinuierlich oder diskontinuierlich, vorzugsweise kontinuierlich mit den Edukten Wasser und C₂,- oder C₃- Olefin beschickt. Das Verhältnis von Wasser zu Olefin mit welchem die Edukte in den Reaktor gefahren werden wird auf ein Molverhältnis von 0,1 bis 0,8, vorzugsweise von 0,15 bis 0,5, eingestellt, Das Einstellen des Molverhältnisses kann z.B. mit Hilfe von Massendurchflußreglern geschehen. Beide Edukte können flüssig oder gasförmig, vorzugsweise gasförmig, in den Reaktor gefahren werden. Zum Verdampfen des Wassers bzw zum Heizen beider Edukte auf Reaktionstemperatur kann es vorteilhaft sein, beide Edukte über eine Verdampferund/oder Thermostatisierstrecke, die elektrisch oder mittels Wärmeträgern auf Reaktionstemperatur beheizt sind, in den Reaktor zu führen. Die Temperatur im Reaktor und die Temperatur, mit welcher die Edukte in den Reaktor strömen, beträgt 160 bis 300 °C. Bei der Hydratisterung von Ethen zu Ethanol beträgt die Temperatur im Reaktor und die Temperatur, mit welcher die Edukte in den Reaktor strömen, vorzugsweise 220 bis 260 °C. Der Druck im Reaktor wird im Bereich von 20 bis 200 bar_{absolut}, vorzugsweise von 60 bis 80 bar_{absolut}, eingestellt.

Der Reaktorausgang ist vorzugsweise mit einem Kühler verbunden, der einen Großteil der unterkritischen Komponenten auskondensiert und diese weiteren Aufarbeitungsschritten zuführt.

Zur Kontrolle der Aktivität und Selektivität des mit Säure getränkten Katalysatorträgers kann es vorteilhaft sein, den Austrittsstrom aus dem Reaktor zu analysieren. Die Analyse kann z.B. gaschromatographisch erfolgen.

Zur Verlängerung der Standzeit des Katalysators kann es vorteilhaft sein kontinuierlich oder diskontinuierlich, vorzugsweise kontinuierlich, die Säure, vorzugsweise Phosphorsäure, mit welcher der katalysatorträger getränkt wurde, in den Reaktor einzubringen. Das Einbringen der Säure in den Reaktor kann z.B. durch Eindüsen erfolgen. Die Menge der Säure, die in den Reaktor eingebracht wird, kann von dem Analyseergebnis des Austrittsstroms abhängig gemacht werden. Die Analyse des Austrittstroms und die Bestimmung der daraus resultierenden Säuremenge die zugegeben wird kann automatisch erfolgen.

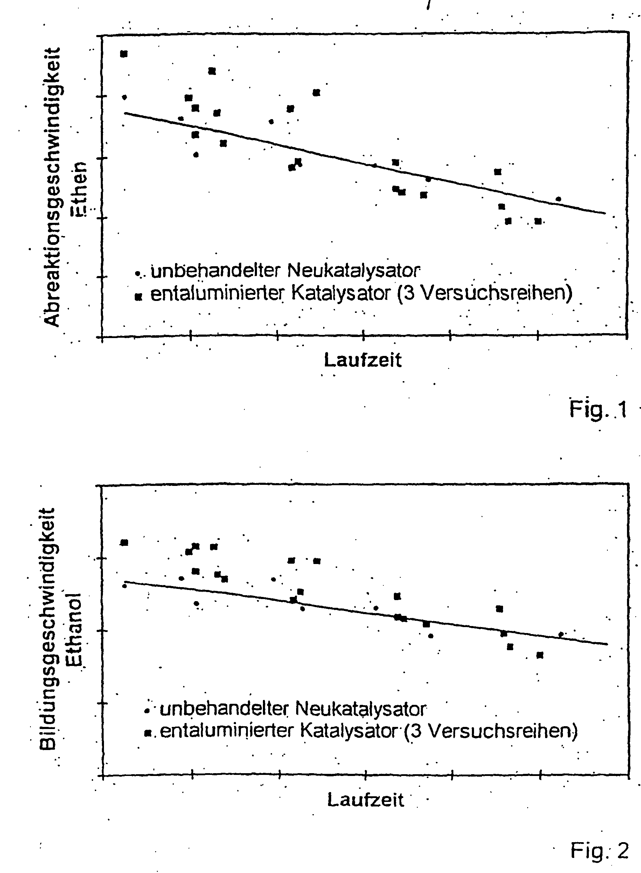

In Figur 1 und 2 sind Abreaktionsgeschwindigkeiten für Ethen sowie Bildungsgeschwindigkeiten für Ethanol bei Verwendung unterschiedlich behandelter Katalysatorträger in Abhängigkeit von der Versuchslaufzeit dargestellt, ohne daß das erfindungsgemäße Verfahren auf diese Ergebnisse beschränkt ist.

### Figur 1

In Fig. 1 ist die Abreaktionsgeschwindigkeit von Ethen bei einer Hydratisierungsreaktion in Abhängigkeit von der Versuchslaufzeit dargestellt. Es sind die Meßwerte von vier Versuchen abgebildet. Die mit Kreisen gekennzeichneten Meßwerte geben die Abreaktionsgeschwindigkeit von Ethen in Bezug auf die Laufzeit wieder, wenn ein Neukatalysatorträger mit dem ursprünglichen Gehalt an Aluminium verwendet wird. Die als Vierecke dargestellten Meßpunkte der Abreaktionsgeschwindigkeit resultieren aus drei Versuchsreihen, die unter Verwendung eines Katalysatorträgers mit verringertem Aluminiumgehalt durchgeführt wurden.

### Figur 2

In Fig. 2 ist die Bildungsgeschwindigkeit von Ethanol bei einer Hydratisierungsreaktion in Abhängigkeit von der Laufzeit der Versuche dargestellt. Es sind die Meßwerte von vier Versuchen abgebildet. Die mit Kreisen gekennzeichneten Meßwerte geben die Bildungsgeschwindigkeit von Ethanol in Bezug auf die Laufzeit wieder, wenn ein Neukatalysatorträger mit dem ursprünglichen Gehalt an Aluminium verwendet wird. Die als Vierecke dargestellten
Meßpunkte der Bildungsgeschwindigkeit von Ethanol resultieren aus drei Versuchsreihen, die unter Verwendung eines Katalysatorträgers mit verringertem Aluminiumgehalt durchgeführt wurden.

Das erfindungsgemäße Verfahren wird anhand der folgenden Beispiele beschrieben, ohne darauf beschränkt zu sein.

### Beispiel 1: Ethanolsynthese mit einem unbehandelten Katalysatorträger

Der Versuch erfolgte in einer Pilotanlage, die als Kernstück einen isotherm betriebenen Rohrreaktor von 1 000 mm Länge und 48 mm Durchmesser besitzt.

Die Edukte Wasser und Ethen werden über eine Verdampfer- bzw. Thermostatisierstrecke, die elektrisch auf Reaktionstemperatur beheizt ist, dem Reaktor zugeführt. Das Wasser wird über eine Pumpe flüssig zudosiert, während das Ethen aus 130-bar. Stahlflaschen entnommen wird. Der Zustrom eines 0,3:1 Gemisches aus Ethen: und Wasser (Molbasis) wird über Massendurchflußregler eingestellt.

Der Reaktorausgang ist mit einem Kühler verbunden, um den Großteil der unterkritischen Komponenten - im wesentlichen Ethanol, Wasser und Diethyletherauszukondensieren; der Rest gelangt ins Abgas, dessen Volumenstrom mit einer Gasuhr bestimmbar ist. Ein Teil des Abgases wird über einen Bypass-Strom einem Gaschromatographen zugeführt. Der Flüssigaustrag wird ebenfalls gaschromatographisch analysiert.

Die Synthese von Ethanol wurde im vorliegenden Beispiel bei 240 °C und 70 bar_{abs} vermessen. Die Standardtestbedingungen sind in Tabelle 1 zusammengefaßt. Als Katalysator wurde ein unbehandelter Neukatalysatorträger, KA- 1 der Südchemie AG, eingesetzt. Die Trägerkenndaten sind in Tabelle 2 zusammengestellt. Die erzielten Umsatz- und Selektivitätswerte zu Versuchsbeginn sind ebenfalls in Tabelle 2 enthalten.

Zur Bestimmung des Aluminiumgehaltes des Katalysatorträgers wurde dieser vor der Durchführung des Versuches mit einem Atomemissionsspektrometer analysiert, um den Gehält an Aluminium zu bestimmen. Als Atomemissionsspektrometer wurde ein induktivgekoppeltes Plasmaatomemissionsspektrometer (ICP-AES) JY 38+ der Firma ISA Jobin Y von verwendet. Die Ergebnisse der Analyse werden in Tabelle 2 wiedergegeben.

### Beispiel 2: Ethanolsynthese mit einem unbehandelten Altträger

Der Versuch wurde wie in Beispiel 1 beschrieben wiederholt. Es wurde diesmal ein schon einmal für die Katalyse einer Hydratisierungssreaktion benutzter unbehandelter Katalysatorträger (Altträger) als Katalysatorträger verwendet. Wiederum gelten die in Tabelle 1 angegebenen Standard-Testbedingungen. Die Ergebnisse des Versuches sowie die Kenndaten des Katalysatorträgers sind ebenfalls wieder in Tabelle 2 wiedergegeben.

Wie den Werten aus Tabelle 2 entnommen werden kann, verringert sich die spezifische Oberfläche des getränkten Katalysatorträgers nach einmaligen Gebrauch als Katalysator. Ebenfalls wird durch die einmalige Verwendung als Katalysator der Aluminiumgehalt auf ca. ¼ des ursprünglichen Gehalts an Aluminium reduziert. Die restlichen ¾ des ursprünglichen Gehalts an, Aluminium des unbehandelten Neuträgers sind während der Hydratisierungsreaktion aus dem Katalysatorträger herausgewaschen worden. Dieses Aluminium bildet die schwerlöslichen Rückstände, die sich bei anschließenden Aufarbeitungsschritten als hinderlich erwiesen haben.

### Beispiel 3: Ethanolsynthese mit behandeltem Altträger

Der Versuch wurde wie in Beispiel 1 beschrieben wiederholt. Als Katalysatorträger kam ein schon benutzter Altfräger zum Einsatz, dessen Gehalt an Aluminium durch Behandlung nach dem erfindungsgemäßen Verfahren verringert wurde. Wiederum gelten die in Tabelle 1 angegebenen Standard-Testbedingungen. Die Ergebnisse des Versuches sowie die Kenndaten des Katalysatorträgers sind ebenfalls wieder in Tabelle 2 wiedergegeben.

Das Desaktivierungsverhalten eines Katalysatorträgers ohne verringertem Aluminiumgehalt und eines mit verringertem Aluminiumgehalt ist in der Fig. 1 und Fig. 2 dargestellt.

Wie aus Tabelle 2 deutlich wird, ist durch Behandlung des Altträgers mit Hilfe des erfindungsgemäßen Verfahrens der Aluminiumgehalt des Katalysatorträgers auf unter 0,03 Gew.-% reduziert worden. Dieser Wert stellt die Nachweisgrenze des benutzten Atomemissionsspektrometers dar. Die Druckfestigkeit des behandelten Altträgers ist mit 30 N/mm immer noch ausreichend, um eine gute Langzeitstabilität des Katalysatorträgers zu garantieren.

Trotz Behandlung des Katalysatorträgers, und Reduzierung des Aluminiumgehaltes auf unter 0,03 Gew.-% ist der Etylenumsatz und die Ausbeute an Ethanol im Vergleich zum unbehandelten und unbenutzten Katalysatorträger (Neuträger) bzw. zum unbehandelten Altträger gleich gut geblieben, im vorliegenden Versuch sogar leicht verbessert worden.

Wie den Figuren 1 und 2 entnommen werden kann, hat die Verringerung des Aluminiumgehaltes nach dem erfindungsgemäßen Verfahren keinen Einfluß auf die Abreaktionsgeschwindigkeit des Ethens und die Bildungsgeschwindigkeit des Ethanols.

**Tabelle 1**

| **Standard-Testbedingungen** | |
|---|---|
| *Prozeßparameter* | *Wert des Prozeßparameters* |
| Gesamtdruck bei der Reaktion | 70 bar_{abs.} |
| Reakortemperatur (isotherm) | 240 °C |
| GHSV | 21,3 l_{N}/min/l_{Kat} |
| Wasser-zu-Ethylen-Verhältnis | 1,0 : 0,3 mol : mol |
| Trägermaterial | KA-1 (Südchemie) |

**Tabelle 2**

| Eigenschaft (getränkter.Träger) | ***Neuträger*** | ***unbehandelten Altträger*** | ***behandelter Altträger*** |
|---|---|---|---|
| Druckfestigkeit | 20 N/mm | 40 N/mm | 30 N/mm |
| spez Oberfläche (BET) | 20 m²/g | 4 m²/g | 3 m²/g |
| Porenvolumen_{gesamt} | 0,7 ml/g | 0,4 ml/g | 0,4 ml/g |
| Al-Gehalt | 1,3 Gew.-% | 0,31 Gew.-% | <0,03 Gew.-% |
| Si-Gehalt | 25 Gew.-%. | 25 Gew.-% | 24 Gew.-% |
| H₃PO₄-Gehalt | 35 Gew.-%. | 36 Gew.-% | 35 Gew.-% |
| Ethylenumsatz zu Versuchsbeginn | 5% | 5% | 6% |
| Raum-Zeit-Ausbeute (Ethanol) zu Versuchsbeginn | 77,4 g/lₖₐₜ/h | 76,4 g/lₖₐₜ/h | 79,8 g/lₖₐₜ/h |

## Patentansprüche

1. Verfahren zur Herstellung eines Katalysators / Katalysatorträgers mit einem Aluminiumgehalt von kleiner 0,03 Gew.% durch Aussetzen von im Wesentlichen Schichtsilikaten, die Aluminium enthalten, folgenden Schritte, beinhaltend Entaluminieren:
- Tränken mit einer Säure,
- hydrothermale Behandlung bei einer Temperatur von 160 bis 300°C und / oder bei einem Wasserdampfpartialdruck von 4 bis 80 bar_{absolut,}
- Waschen mit saurer, basischer oder neutraler Lösung
- sowie ggf. Nachwaschen mit Wasser,
wobei der Schritt des Tränkens mit einer Säure, das Tränken mit Phosphorsäure umfasst.

## Claims

1. Method for producing a catalyst/catalyst support with an aluminum content of less than 0.03 wt% by exposing essentially phyllosilicates containing aluminum, to the following steps, comprising dealumination:
- Impregnating with an acid,
- Hydrothermal treatment at a temperature of 160 to 300°C and/or at a water vapor partial pressure of 4 to 80 bar_{absolute},
- Washing with acidic, alkaline or neutral solution
- And optionally rewashing with water,
wherein the step of impregnating with an acid comprises impregnating with phosphoric acid.

## Revendications

1. Procédé de production d'un catalyseur/support de catalyseur avec une teneur en aluminium inférieure à 0,03 % en poids par exposition principalement de phyllosilicates contenant de l'aluminium, aux étapes suivantes, comprenant la désalumination :
- imprégnation avec un acide,
- traitement hydrothermal à une température de 160 à 300 °C et/ou à une pression partielle de vapeur d'eau comprise entre 4 et 80 bars_{absolus},
- lavage dans une solution acide, alcaline ou neutre
- et éventuellement relavage à l'eau,
l'étape d'imprégnation par un acide comprenant l'imprégnation par de l'acide phosphorique.
